# EUROPEAN PATENT APPLICATION

(11) **EP 3 854 454 A1**
(43) Date of publication of application: **28.07.2021**
(21) Application number: 18938737.6
(22) Date of filing: 05.11.2018
(51) Int. Cl.: A61N 7/02, A61B 18/04, A61N 7/00

(54) **BODY CAVITY INSERTABLE ULTRASOUND APPARATUS HAVING STRUCTURE FOR CIRCULATION OF ULTRASOUND TRANSMISSION MEDIUM**

(30) Priority: 01.11.2018 KR 20180133151
(71) Applicant: Korust Co., Ltd., Anyang-si, Gyeonggi-do 14056 (KR)
(72) Inventor: CHO, Sung-Chan, Gunpo-Si Gyeonggi-do 15851 (KR); LEE, Jung Woo, Seoul 08070 (KR); JEONG, Hyun Du, Busan 48736 (KR); BANG, Jin Hyun, Seoul 03102 (KR)
(74) Representative: Manitz Finsterwald Patent- und Rechtsanwaltspartnerschaft mbB
(86) International application number: PCT/KR2018/013296
(87) International publication number: WO 2020/091125

(57) **Abstract**

An ultrasound apparatus of a body cavity insertable type includes: a handpiece; a supporting rod that is elongated from the handpiece; an ultrasound probe that includes a housing that is connected to the supporting rod and an ultrasound transducer that is fixed to the housing; and an ultrasound transmission medium circulation mechanism that is configured to discharge an ultrasound transmission medium filled in an ultrasound transmission space that is formed at a front of the ultrasound transducer and to inflow the discharged ultrasound transmission medium to the ultrasound transmission space again. The housing and the ultrasound transducer are configured such that the ultrasound transmission medium filled in the ultrasound transmission space is prevented from inflowing to a rear space of the ultrasound transducer.

## Description

### [Technical Field]

The present invention relates to an ultrasound apparatus of a body cavity insertable type which can be inserted into a body cavity such as a nasal cavity, an oral cavity, a pharynx, a vagina or the like and can perform an ultrasound treatment.

### [Background Art]

An ultrasound is used for various treatments and surgical procedures, and as an example a treatment method of treating diseases by noninvasively irradiating ultrasound in the tissues of a body cavity such as a nasal cavity has been introduced. A conventional ultrasound apparatus of a nasal cavity insertable type has a rod type supporting member which can be inserted into the nasal cavity and an ultrasound probe which is mounted on an end portion thereof. The ultrasound probe irradiates ultrasound in a state of being inserted into the nasal cavity, so a heat lesion is formed in the tissues within a nose in a noninvasive way to cure a disease such as a rhinitis.

Such an ultrasound apparatus of a nasal cavity insertable type has not yet been widely used, and technical progresses in various aspects such as an efficiency of an ultrasound irradiation, a stable structure and the like are required. In particular, an ultrasound apparatus of a body cavity insertable type requires that a front space of an ultrasound transducer should be filled with ultrasound transmission medium for ultrasonic transmission, and also requires a measure for radiating heat generated in an ultrasound apparatus because heat generated in an ultrasound apparatus which is used in a state of being inserted into a body cavity inevitably cause side effects.

### <Prior Art Document>

1. Korean Patent Registration No. 10-1857026
2. Korean Patent Registration No. 10-1896565
3. Korean Patent Registration No. 10-1886673
4. US Patent Publication No. US2008/0027423

### [Detailed Description of the Invention]

### [Technical Problem]

An object to be solved by the present invention is to provide an ultrasound apparatus of a body cavity insertable type that is capable of smooth supply of an ultrasonic transmission medium filled in a front space of an ultrasonic transducer, discharge of air bubbles, and discharge of heat generated inside an ultrasound apparatus.

### [Technical Solution]

An ultrasound apparatus of a body cavity insertable type according to an embodiment of the present invention includes: a handpiece; a supporting rod that is elongated from the handpiece; an ultrasound probe that includes a housing that is connected to the supporting rod and an ultrasound transducer that is fixed to the housing; and an ultrasound transmission medium circulation mechanism that is configured to discharge an ultrasound transmission medium filled in an ultrasound transmission space that is formed at a front of the ultrasound transducer and to inflow the discharged ultrasound transmission medium to the ultrasound transmission space again. The housing and the ultrasound transducer are configured such that the ultrasound transmission medium filled in the ultrasound transmission space is prevented from inflowing to a rear space of the ultrasound transducer.

The housing may be configured to support entirely an edge of the ultrasound transducer so as to fluidically separate a front space and a rear space of the ultrasound transducer.

The housing may be configured to support entirely lateral edges of the ultrasound transducer and longitudinal edge of the ultrasound transducer are sealed by sealing material, in order to fluidically separate a front surface and a rear surface of the ultrasound transducer.

Lateral edges of the ultrasound transducer may be formed to have a concave curved surface shape, and the housing may have a supporting portion having a shape corresponding to the concave curved surface of the lateral edge so as to support the lateral edge.

The ultrasound transmission medium circulation mechanism includes: a reservoir storing the ultrasound transmission medium; a pump that is configured to move the ultrasound transmission medium from the reservoir to the ultrasound transmission space; an inlet passage forming a passage through which the ultrasound transmission medium discharged from the reservoir inflows into the ultrasound transmission space; and an outlet passage forming a passage through which the ultrasound transmission medium discharged from the ultrasound transmission space inflows into the reservoir.

An ultrasound apparatus of a body cavity insertable type according to another embodiment of the present invention further includes a sealing member that is disposed between the ultrasound probe and the supporting rod. The inlet passage and the outlet passage may pass through the sealing member.

The inlet passage may include an inlet conduit passing through the supporting rod and the outlet passage may include an outlet conduit passing through the supporting rod. The inlet conduit and the outlet conduit may pass through the sealing member.

The inlet passage may include an inlet conduit passing through the supporting rod and the outlet passage comprises an outlet conduit passing through the supporting rod. The housing may include: an inlet connection passage that is connected to the inlet conduit; an inlet hole that connects the inlet connection passage and the ultrasound transmission space; an outlet connection passage that is connected to the outlet conduit; and an outlet hole that connects the outlet connection passage and the ultrasound transmission space.

The inlet connection passage and the inlet hole may be disposed at one side with respect to a longitudinal direction of the hosing, and the outlet connection passage and the outlet hole may be disposed at the other side with respect to the longitudinal direction to face respectively the inlet connection hole and the inlet hole.

The reservoir may have a window for checking an amount of the ultrasound transmission medium filled therein.

The reservoir may have an ultrasound transmission medium injection port for replenishing the ultrasound transmission medium and an automatic closing valve that is installed in the ultrasound transmission medium injection port.

The reservoir may be fixedly provided outside the handpiece, and the reservoir may include an inlet through which the ultrasound transmission medium discharged from the ultrasound probe inflows therein and an outlet through which the ultrasound transmission medium to be supplied to the ultrasound probe is discharged. The outlet may be disposed lower than the outlet.

The ultrasound apparatus of a body cavity insertable type according to another embodiment of the present invention may further include a gyro sensor that detects a posture of the handpiece and a controller that controls an operation of the pump based on a signal of the gyro sensor. The reservoir is installed within the handpiece. The controller may control such that an alarm signal is output or control to operate the pump so that the ultrasound transmission space is filled with the ultrasound transmission medium.

The ultrasound apparatus of a body cavity insertable type of according to yet another embodiment of the present invention may further include a gyro sensor that detects a posture of the handpiece and a controller that controls an operation of the pump based on a signal of the gyro sensor. The reservoir may be disposed within the handpiece. The housing may include an inlet hole through which the ultrasound transmission medium inflows into the ultrasound transmission space and an outlet hole through which the ultrasound transmission medium is discharged from the ultrasound transmission space. In case that based on a signal of the gyro sensor, the handpiece is in a position in that the ultrasound transmission medium is full to an outlet of the reservoir and the outlet hole faces upward, the controller may control such that an alarm signal is output or control to operate the pump so that the ultrasound transmission space is filled with the ultrasound transmission medium to discharge air bubbles remaining in the ultrasound transmission space.

The ultrasound apparatus of a body cavity insertable type according to yet another embodiment of the present invention may further include a gyro sensor that detects a posture of the handpiece and a controller that controls an operation of the pump based on a signal of the gyro sensor. The housing may include an inlet hole through which the ultrasound transmission medium inflows into the ultrasound transmission space and an outlet hole through which the ultrasound transmission medium is discharged from the ultrasound transmission space. In case that based on a signal of the gyro sensor, the handpiece is in a position in that the outlet hole faces upward, the controller may control such that an alarm signal is output or control to operate the pump so that the ultrasound transmission space is filled with the ultrasound transmission medium to discharge air bubbles remaining in the ultrasound transmission space.

The housing may be provided with an ultrasound passing hole through which a generated ultrasound passes, and the ultrasound apparatus may further include a sealing cover that seals the ultrasound passing hole.

The sealing cover may include: a cover member that is configured to be put on the housing and has an ultrasound passing window that is formed at a position corresponding to the ultrasound passing hole; and an ultrasound penetration film that is attached to the cover member to seal the ultrasound passing hole.

### [Effect of the Invention]

According to the present invention, since the ultrasound transmission medium filled in the ultrasound transmission space of the ultrasound probe circulates, cooling and discharging air bubbles can be obtained.

In addition, since the front space and the rear space of the ultrasound transducer are fluidically separated from one another, the front surface and the rear surface of the ultrasound transducer can be electrically separated from one another.

### [Brief Description of the Drawings]

FIG. 1 is a perspective view of an ultrasound apparatus of a body cavity insertable type according to an embodiment of the present invention.
FIG. 2 is an exploded perspective view of a supporting rod, an ultrasound probe and a sealing cover of an ultrasound apparatus of a body cavity insertable type according to an embodiment of the present invention.
FIG. 3 is an exploded bottom perspective view of a supporting rod, an ultrasound probe and a sealing cover of an ultrasound apparatus of a body cavity insertable type according to an embodiment of the present invention.
FIG. 4 is a sectional view taken along a line IV-IV in FIG. 3.
FIG. 5 is a sectional view taken along a line V-V in FIG. 3.
FIG. 6 is a sectional view taken along a line VI-VI in FIG. 3.
FIG. 7 is a sectional view taken along a line VII-VII in FIG. 3.
FIG. 8 shows a reservoir and a pump of an ultrasound apparatus of a body cavity insertable type according to an embodiment of the present invention.
FIG. 9 shows a reservoir and a pump of an ultrasound apparatus of a body cavity insertable type according to another embodiment of the present invention.

### [Detailed Description of Embodiments]

Embodiments of the present invention will be described in detail with reference to the accompanying drawings hereinafter.

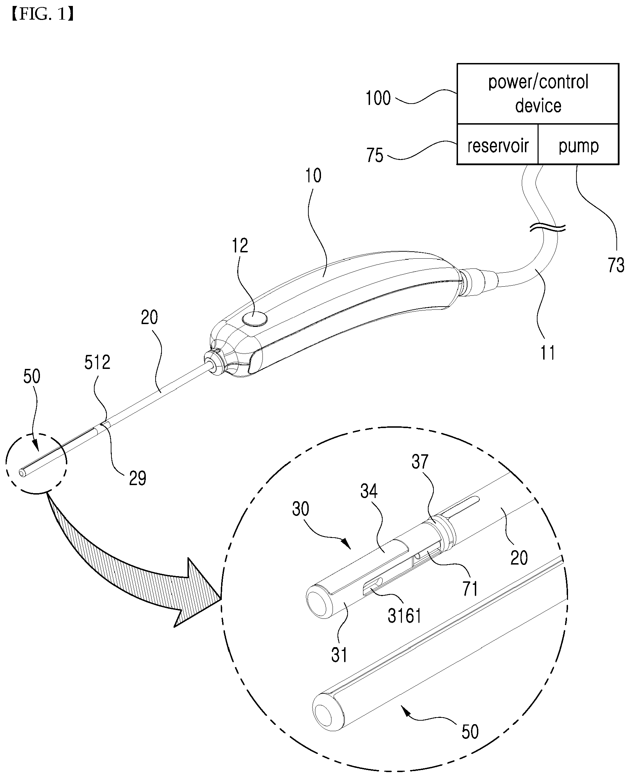

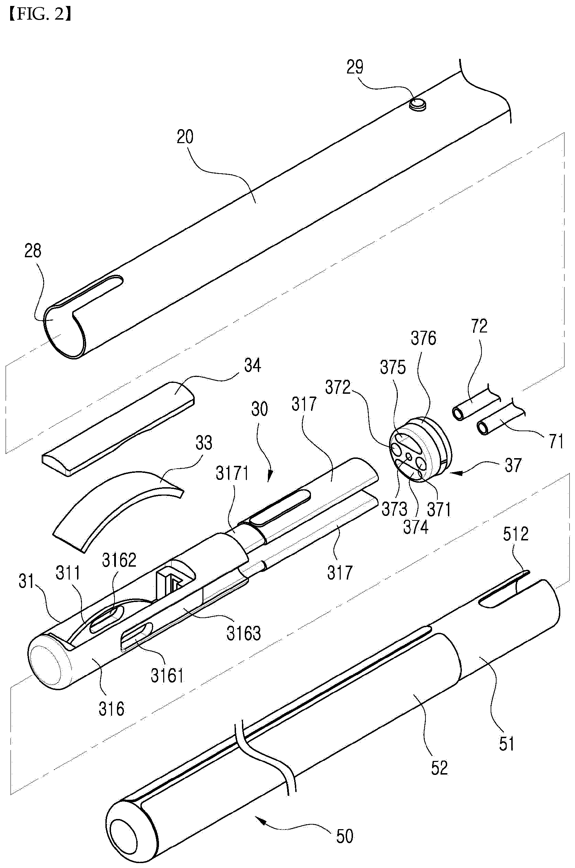

An ultrasound apparatus of a body cavity insertable type according to an embodiment of the present invention is configured to be able to irradiate ultrasound in a state of being inserted into a body cavity such as a nasal cavity, an oral cavity, a pharynx, and a vagina to make heat lesion in the tissues. Referring to FIG. 1 and FIG. 2, an ultrasound apparatus according to an embodiment of the present invention includes a handpiece 10, a supporting rod 20, an ultrasound probe 30 and a separable sealing cover 50.

The handpiece 10 may be formed to be able to be grasped by a hand of an operator performing an ultrasound treatment. As exemplarily shown in FIG. 1, the handpiece 10 may be configured to be connected to an external power/control device 100 via an electric power cable 11 and may be provided with an on/ off button 12 to regulate an ultrasound generation by electric power on/off. The power/control device 100 may be configured to provide electric power for the generation of ultrasound and various controls for ultrasound treatment. The handpiece 10 may be separately formed and may be connected to the electric power cable 11 or may be formed as a separate part including the electric power cable 11 and may be connected to the power/control device 100.

The supporting rod 20 may be extended from the handpiece 10 and may have a shape of an elongated rod. Since a distal end portion of the supporting rod 20 may be inserted into the nasal cavity or the like during an ultrasound treatment, the supporting rod 20 is formed as a rod shape having a relatively small diameter which can be inserted into the body cavity. As shown in FIG. 1, the supporting rod 20 is extended from a distal end of the handpiece 10. At this time, the supporting rod 20 may be formed as a part separated from the handpiece 10 to be connected to the handpiece 10 or may be formed integrally with the handpiece 10.

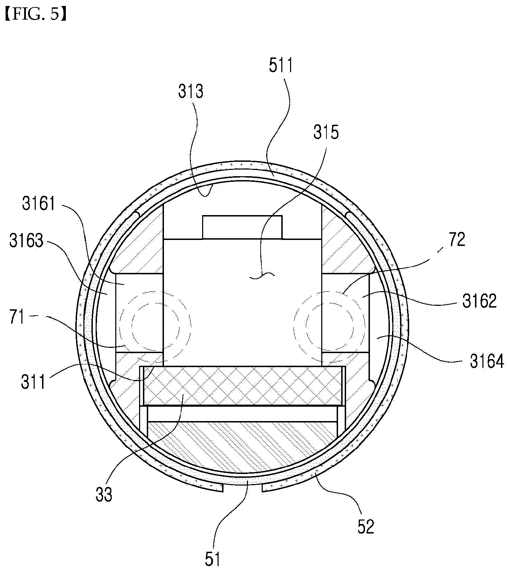

The ultrasound probe 30 causes an ultrasound vibration due to the application of a pulsed electric power. Referring to FIG. 2 and FIG.5, the ultrasound probe 30 includes a housing 31 and an ultrasound transducer 33. The ultrasound transducer 33 may be formed by forming a pair of electrodes on both sides of a piezoelectric material layer such as a piezoelectric ceramic layer as well known in the art and is configured such that the piezoelectric material layer vibrates when a pulsed electric power is applied to both electrodes. Not shown in the drawings, electric cables may be provided to apply the pulsed electric power to the electrodes of the ultrasound transducer 33. For example, the electric cables may be electrically connected to the electrodes of both sides of the ultrasound transducer 33 after passing through the handpiece 10, the supporting rod 20 and the housing 31.

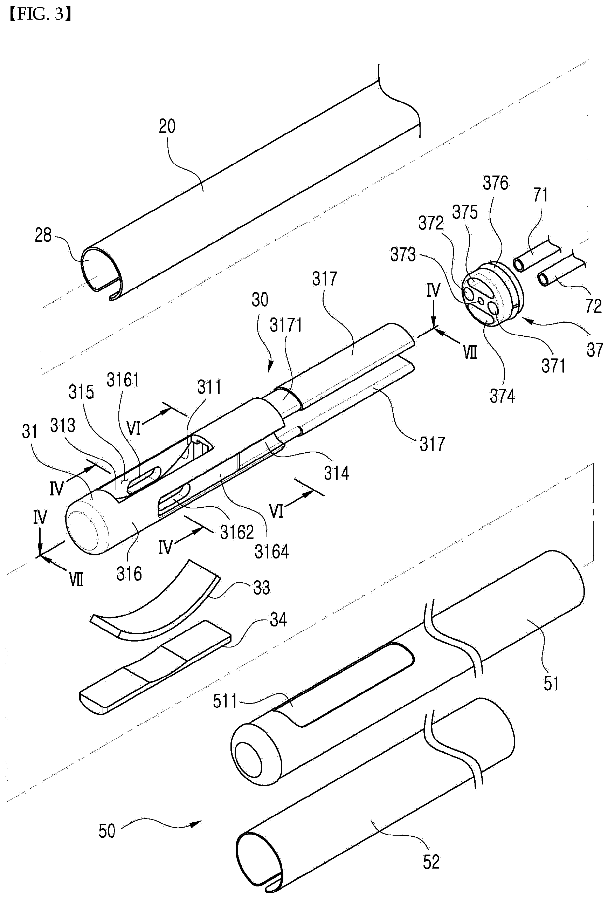

The housing 31 of the ultrasound probe 30 is connected to the supporting rod 20 and is configured to be able to be inserted into the body cavity such as the nasal cavity. The housing 31 may include an ultrasound transducer mounting portion 316 to which the ultrasound transducer 33 is mounted and an insertion portion 317 for the connection with the supporting rod 20. At this time, the ultrasound transducer mounting portion 316 may form a mounting space 315 into which the piezoelectric element 33 is disposed, and a frontal space of the ultrasound transducer 33, i.e., an ultrasound transmitting space may be filled with an ultrasound transmission medium. For example, as shown in FIG. 3, the supporting rod 20 may be formed as a hollow tube having an insertion hole 28 which is elongated along a longitudinal direction. The insertion portion 317 of the housing 31 is formed to have a diameter slightly less than an inner diameter of the insertion hole 28 and may be fitted into the insertion hole 28 of the supporting rod 20. At this time, according to an embodiment of the present invention, a diameter of the ultrasound transducer mounting portion 316 may be greater than the diameter of the insertion portion 317. Accordingly, the size of the ultrasound transducer 33 can be increased by the increase of the ultrasound transducer mounting portion 316, and thus the ultrasound energy can be increased.

The supporting rod 20 may be formed of metal in order to secure a required durability and a structural strength, and the housing 31 may be formed of electrical insulation material such as synthetic resin for electric insulation from the piezoelectric element 33.

The housing 31 forms an ultrasound passing hole 313 through which ultrasound generated by the ultrasound transducer 33 passes. For example, ultrasound generated by the ultrasound transducer 33 progress into tissues forming the body cavity after having passed the ultrasound passing hole 313.

The ultrasound transducer 33 may be disposed to irradiate ultrasound in a direction perpendicular to the longitudinal direction of the housing 31. Accordingly, in a state of being inserted into the body cavity, the ultrasound transducer 33 can easily irradiate ultrasound into tissues of the body cavity. For this, the ultrasound passing hole 313 of the housing 31 may be formed to face in a direction perpendicular to the longitudinal direction.

The housing 31 may form an ultrasound transmission space 315 which is connected to the ultrasound passing hole 313 and is filled with the ultrasound transmission medium. As shown in FIG. 2 and FIG. 3, one surface of the ultrasound transducer 33 is exposed to the ultrasound transmission space 315, and the ultrasound transmission space 315 is filled with the ultrasound transmission medium of liquid state. For example, the ultrasound transmission medium may be water.

Referring to FIG. 2 and FIG. 3, the ultrasound transducer 33 may be formed to have a curvature about a direction perpendicular to the longitudinal direction of the supporting rod 20. That is, the curved surface of the ultrasound transducer 33 is elongated in a longitudinal direction of the supporting rod 20, i.e., in an insertion direction into the body cavity. Due to this configuration the structure of the ultrasound probe 30 of a ling rod shape which can be inserted into the body cavity can be realized. The ultrasound transducer 33 may have a concave shape, a concave shape obtained by removing both sides of a concave surface, a cylindrical surface, a bended cylindrical surface which is obtained by bending inwardly both side portions of a cylindrical surface, or a shape consisting of a plurality of same shapes.

According to an embodiment of the present invention, a sealing mechanism for sealing the ultrasound transmission medium in a liquid state such as water that is filled in the ultrasound transmission space 315 is provided. That is, the sealing mechanism prevents the ultrasound transmission medium from leaking through the ultrasound passing hole 313 that is formed in the housing 31 for propagation of ultrasound. For example, the sealing mechanism may be formed as a sealing cover 50 that is covered with the housing 31.

Referring to FIG. 1 to FIG. 4, the sealing cover 50 is separably connected to the ultrasound probe 30 so as to at least partially enclose the ultrasound probe 30 from a distal end thereof. The ultrasound treatment may be performed in a state of coupling the sealing cover 50 to the ultrasound probe 30.

The sealing cover 50 may include a cover member 51 having a tube shape one end of which is closed. The cover member 51 may have an ultrasound passing window 511 that is formed at a position corresponding to the ultrasound passing hole 313 of the housing 31. In case that the ultrasound transmission space 315 is filled with an ultrasound transmission medium of a liquid state, an ultrasound penetration film 52 for sealing the ultrasound passing window 511 may be attached to the cover member 51 or may be put on the cover member 51. For example, the ultrasound penetration film 52 may be attached to an outer surface of the cover member 51 by a double-sided adhesive tape. Meanwhile, in another embodiment, the ultrasound penetration film 52 may be formed in a type of a condom which is mad of thin film, and by putting the ultrasound penetration film 52 on the cover member 51, the ultrasound passing window 511 can be sealed.

A sealing structure may be formed in order to prevent the ultrasound transmitting medium from leaking through a gap between the housing 31 and the sealing cover 50 when the sealing cover 50 is connected to the housing 31. According to an embodiment of the present invention, the sealing structure may include an annular sealing protrusion 376 that is formed on the sealing member 37. The annular sealing protrusion 376 may be formed by being radially outwardly protruded and tightly contacts and contacts an inner circumferential surface of the cover member 51 of the sealing cover 50 in a state that the sealing cover 50 is coupled. Accordingly, the ultrasound transmission medium filled with the ultrasound transmission space 115 can be prevented from leaking to the outside.

In order to allow the cover member 51 to be inserted into the correct position, a guide indentation 512 is formed in the cover member 51, and a guide protrusion 29 that is inserted into the guide indentation 512 is formed in the supporting rod 20. The cover member 51 can be inserted into a predetermined position by covering the cover member 51 with the guide protrusion 29 inserted into the guide indentation 512.

The housing 31 and the ultrasound transducer 33 are configured such that the ultrasound transmission medium filled in the ultrasound transmission space 315 does not flow into the space behind the ultrasound transducer 33. That is, the front space and the rear space of the ultrasound transducer 33 are fluidly blocked from each other so that both electrodes of the ultrasound transducer 33 are prevented from being shortcircuited by the ultrasound transmission medium.

According to an embodiment of the present invention, the housing 31 may be configured to support the entire periphery of the widthwise edges the lengthwise edges of the ultrasound transducer 33. At this time, the entire periphery of the ultrasound transducer 33 may be bonded by adhesive to thereby be sealed.

As shown in FIG. 2 to FIG. 5, the housing 31 of the ultrasound probe 30 may have a substantially cylindrical shape and is formed such that the ultrasound transducer 33 is mounted thereto. The ultrasound transducer 33 may be attached to the housing 31 by adhesive. For example, the ultrasound transducer 33 may have a cylindrical surface or a deformed cylindrical surface both ends of which are bended, and the housing 31 may have a seating surface 311 that has a shape corresponding to the shape of the widthwise edge of the ultrasound transducer 33. As shown in FIG. 5, a pair of the seating surfaces 311 may be disposed to face each other, and both edges of the ultrasound transducer 33 having a curvature are entirely supported on the pair of the seating surfaces 311. In addition, lengthwise edges of the ultrasound transducer 33 are also supported on seating surfaces provided in the housing 31.

The entire of the ultrasound transducer 33 is supported by the seating surface of the housing 31 so that the sealing can be obtained. Further, because both edges of the ultrasound transducer 33 having a curvature are seated in contact with the seating surfaces 311 of the housing 31, the ultrasound transducer 33 can be stably supported even while it vibrates for a long time. In particular, if ultrasound is generated for a long time without supporting both ends of the ultrasound transducer 33, the ultrasound transducer 33 can be destroyed near the adhesive surface. Such a destruction is caused by the principle that the ultrasound transducer 33 generates ultrasound by repeated minute displacements thereof. In order to prevent the destruction of the ultrasound transducer, in an embodiment of the present invention, the seating surfaces 311 that support both edges of the ultrasound transducer 33, and both edges of the ultrasound transducer 33 are bonded with adhesive in a close contact with the seating surfaces 311. In particular, when the ultrasound transducer has a narrow width and a long length in an ultrasound apparatus of a body cavity insertable type, the lateral edges of the ultrasound transducer 33 needs to be supported.

Meanwhile, according to another embodiment of the present invention, edges in the width direction of the ultrasound transducer are supported on the seating surface of the housing, and edges in the length direction are in close contact with an inner surface of the housing and may be sealed by a sealing material such as silicone.

The housing 31 may include an installation hole 314 that is formed by removing a portion facing the rear of the ultrasound transducer 33, and a rear cover 34 may be provided to cover this portion in a state that the ultrasound transducer 33 is installed. When the ultrasound transmission medium penetrates through the rear of the housing 31 to which the ultrasound transducer 33 is attached, the ultrasound transducer 33 may be damaged or a defect may occur due to an electrical short of the circuit. In order to prevent this, a separate rear cover 34 is coupled to prevent the penetration of the ultrasound transmission medium to the rear surface of the ultrasound transducer 33 to achieve the sealing.

An ultrasound apparatus of a body cavity insertable type according to an embodiment of the present invention includes an ultrasound transmission medium circulation structure 70 for circulating the ultrasound transmission medium filled in the ultrasound transmission space 315. The ultrasound transmission medium circulation structure 70 acts to discharge the ultrasound transmission medium filled in the ultrasound transmission space 315 that is formed by the housing 31 and the ultrasound transducer 33 to the outside of the ultrasound transmission space 315 and then to flow into the ultrasound transmission space 315. The ultrasound transmission medium containing the heat inside the ultrasound transmission space 315 is discharged outside to emit the heat, so that the ultrasound probe 30 can be cooled. In addition, it is possible to prevent the shortage of the ultrasound transmission medium in the ultrasound transmission space due to the circulation of the ultrasound transmission medium, and when bubbles, air or the like other than liquid are generated in the ultrasound transmission space, it can be discharged together when the ultrasound transmission medium is circulated.

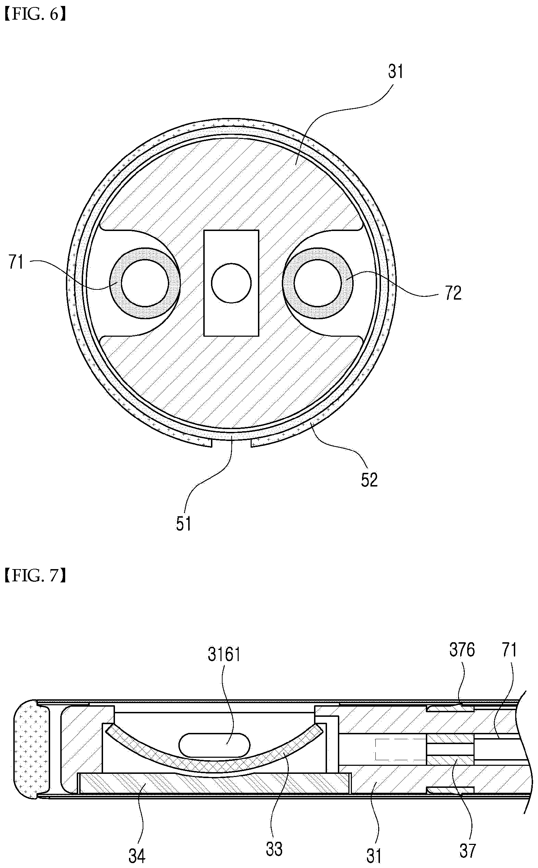

Referring to FIG. 2 to FIG. 7, the ultrasound transmission medium circulation structure 70 may include an outlet conduit 71 for discharging the ultrasound transmission medium in the ultrasound transmission space 315, an inlet conduit 72 for flowing the discharged ultrasound transmission medium into the ultrasound transmission space 315 again, a pump 73 providing a force to induce the flow of the ultrasound transmission medium and a reservoir 75 for storing the ultrasound transmission medium. The ultrasound transmission medium may be water and the pump 73 may be a known water pump capable of pumping water. Although FIG. 1 illustrates an example in which the pump 73 and the reservoir 75 are disposed outside the handpiece 10, the pump 73 and the reservoir 75 may be disposed within the handpiece 10. The outlet conduit 71 and the inlet conduit 72 may be formed as a tube member in the form of a hose or is implemented as a passage in the supporting rod 20.

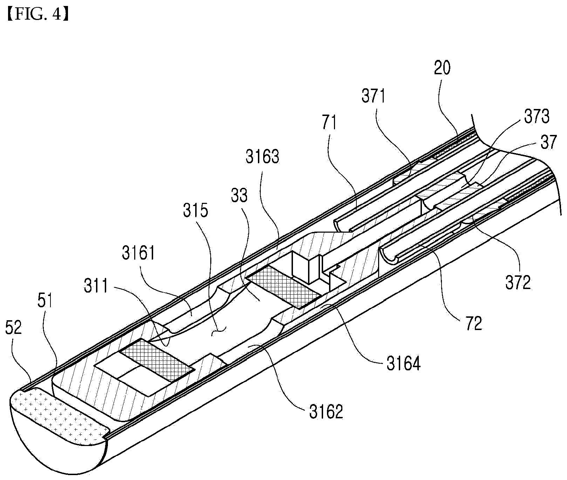

Referring to FIG. 2 to FIG. 4, the housing 31 may include an outlet hole 3161 and an inlet hole 3162 for an outlet and an inlet of the ultrasound transmission medium of a liquid state filled in the ultrasound transmission space 315, and the ultrasound transmission space can be fluidically connected to the reservoir 75 containing the ultrasound transmission medium via the inlet conduit 72 and the outlet conduit 71 that are respectively fluidically connected to the inlet hole 3162 and the outlet hole 3161. At this time, as shown in FIG. 4, the inlet conduit 72 and the inlet hole 3162 may be connected to one another by an inlet connection passage 3164, and the outlet hole 3161 and the outlet conduit 71 can be connected to one another by an outlet connection passage 3163. The inlet and outlet connection passages 3164 and 3163 may be formed as an indented space at the side of the housing 31 and can be surrounded by the sealing cover 50 to be sealed.

A sealing member 37 for preventing the leakage of the ultrasound transmission medium may be disposed between the supporting rod 20 and the ultrasound probe 30. The inlet conduit 71 and the outlet conduit 71 may be installed to pass through the sealing member 37. In order to increase the sealing effect, the sealing member 37 may be formed of rubber or silicone. In addition, the inlet conduit 72 and the outlet conduit 71 pass through the sealing member 37 and the sealing member 37 is fitted to an inner circumferential surface of the supporting rod 20, so that the ultrasound transmission medium filled in the housing can be prevented from inflowing into the supporting rod 20.

Referring to FIG. 2 and FIG. 3, the sealing member 37 may have through holes 372 and 371 through which the inlet conduit 72 and the outlet conduit 71 pass respectively, and the inlet conduit 72 and the outlet conduit 71 that pass through the supporting rod 20 are inserted into the through holes 372 and 371 of the sealing member 37 to be fluidically connected to the inlet and outlet holes 3162 and 3161. An insertion portion 317 of the housing 31 can be formed as two parts facing one another in a vertical direction, and the sealing member 37 may have through holes 374 and 375 into which two parts of the insertion portion 317 are inserted. As shown in FIG. 2 and FIG. 3, the insertion portion 317 may have neck portions 3171, and the neck portions 3171 can be positioned in the through holes 374 and 375. In addition, the sealing member 37 may have a though hole 373 through which an electrical power cable (not shown) for applying electrical current to the ultrasound transducer 33. Meanwhile, the housing 31 may have a passage through which the cable having passed the through hole 373 of the sealing member 37, and this passage may be connected to the rear space of the ultrasound transducer 33 to be blocked from the ultrasound transmission medium. The ultrasound transmission medium can be prevented from leaking into the supporting rod 20 by the sealing member 37.

The outlet conduit 71 and the inlet conduit 72 may pass through the handpiece 10 and the supporting rod 20 and may be connected to the ultrasound probe 30. The pump 73 and the reservoir 75 may be separately provided outside, and as exemplarily shown in FIG. 1 may be accommodated together in a case in which the power/control device 100 is accommodated. At this time, the outlet conduit 71, the pump 73, the reservoir 75 and the inlet conduit 72 may be connected to one another so that the ultrasound transmission medium can be circulated.

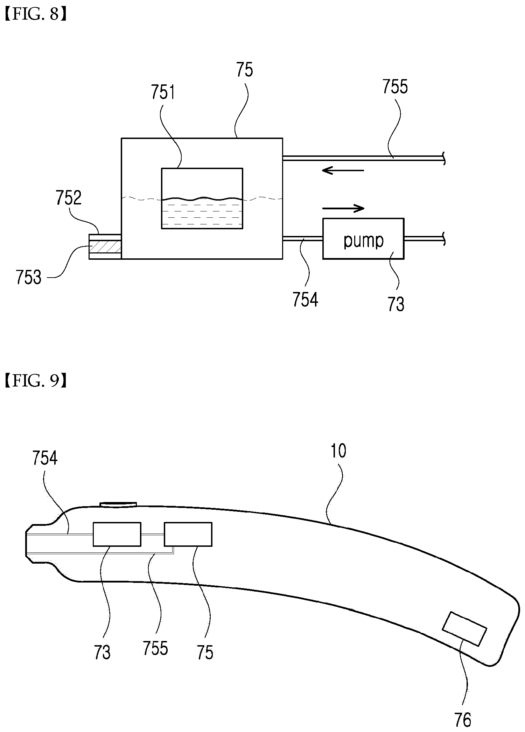

Referring to FIG. 8, the reservoir 75 storing the ultrasound transmission medium may have a window 751 for checking the amount of the ultrasound transmission medium stored therein. The window 751 may formed of a transparent material so that the inside thereof can be seen. Since the amount of the ultrasound transmission medium can be reduced due to vaporization or minute leakage when the ultrasound apparatus is used for a long time, the user can check the amount of the ultrasound transmission medium filled in the reservoir 75 through the window 751 and can add the ultrasound transmission medium when the amount of the ultrasound transmission medium becomes below a certain level.

Meanwhile, the reservoir 75 may have an ultrasound transmission medium injection port 752 for replenishing the ultrasound transmission medium, and an automatic closing valve 753 can be installed in the ultrasound transmission medium injection port 752. For example, the ultrasound transmission medium can be replenished using a syringe-type injection tool, and the automatic closing valve 753 can be configured to be opened when the ultrasound transmission medium is being injected and to be automatically closed after the injection is completed.

Meanwhile, in another embodiment of the present invention, the reservoir may be disposed within the handpiece.

Referring to FIG. 8, an outlet 754 and an inlet 755 for circulation of the ultrasound transmission medium is provided to the reservoir 75. The outlet 754 serves as a passage for supplying the ultrasound transmission medium stored in the reservoir 75 to the ultrasound probe 30, and the inlet 755 serves as a passage for supplying the ultrasound transmission medium of the ultrasound probe 30 to the reservoir 75. That is, the ultrasound transmission medium in the reservoir 75 is discharged through the outlet 754 to flow toward the ultrasound probe 30 in response to an operation of the pump 73, and thereby the ultrasound transmission medium filled in the ultrasound probe 30 is pushed out and is introduced into the reservoir 75 via the inlet 755. The circulation structure of the ultrasound transmission medium is thereby realized. At this time, for a stable supply of the ultrasound transmission medium, the reservoir 75 can be located at the handpiece 10 or a main body, and the outlet 754 is disposed at a lower position than the inlet 755, for example, at a bottom of the reservoir 75 and the pump 73 may be connected to the outlet 754. Accordingly, even when air is present in the reservoir 75, the ultrasound transmission medium locates at the lower portion of the reservoir 75 due to the gravity can be stably supplied to the ultrasound probe 30 through the outlet 754.

Meanwhile, in case that the reservoir 75 is disposed within the handpiece 10 as shown in FIG. 9, by operating the pump 73 in a state that the outlet 754 is positioned downward, air in the reservoir 75 can be prevented from being supplied to the ultrasound probe 30.

At this time, a gyro sensor 76 that detects the posture of the handpiece 10 is installed, and the power/control device 100 may operate to display an alarm signal or to allow the ultrasound transmission medium to be supplied to the ultrasound probe 30 by operating the pump 73 when it is determined that the handpiece 10 is in a state that the ultrasound transmission medium is full to the outlet 754 based on a signal of the gyro sensor 76. The alarm signal may be a lamp, a buzz or the like, and an operator may recognize the alarm signal and then operate the pump to make the ultrasound transmission medium be supplied. The state of the handpiece 10 may be determined based on the signal of the gyro sensor 76, and the circulation of the ultrasound transmission medium may be made or stopped based on the signal.

In addition, it is possible to discharge air bubbles remaining in the ultrasound transmission space 315 using a signal of the gyro sensor 76 that is installed in the handpiece 10. For example, if it is determined that that the handpiece 10 is in a position in that the outlet hole 3161 faces upward, the power/control device 100 operates to output an alarm signal or to operate the pump 73 so that the supplied ultrasound transmission medium discharges air bubbles remaining in the ultrasound transmission space 315 while filing the ultrasound transmission space 315. On the other hand, in another embodiment, if it is determined that the handpiece 10 is in a position in that the ultrasound transmission medium is full to the outlet 754 and at the same time the outlet hole 3161 faces upward, the power/control device 100 operates to output an alarm signal or to operate the pump 73 so that the supplied ultrasound transmission medium discharges air bubbles remaining in the ultrasound transmission space 315 while filing the ultrasound transmission space 315.

Meanwhile, according to another embodiment of the present invention, as shown in FIG. 1, a reservoir storing the ultrasound transmission medium is disposed outside the handpiece, for example, in a separate main body, it is configured that air bubbles remaining in the ultrasound transmission space 315 are discharged using the gyro sensor 76 that is installed in the handpiece 10. As described above, if it is determined that that the handpiece 10 is in a position in that the outlet hole 3161 faces upward, the power/control device 100 operates to output an alarm signal or to operate the pump 73 so that the supplied ultrasound transmission medium discharges air bubbles remaining in the ultrasound transmission space 315 while filing the ultrasound transmission space 315.

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

### [Industrial Applicability]

The present invention relates to an ultrasound apparatus of a body cavity insertable type which can be sued for an ultrasound treatment to have an industrial applicability.

## Claims

1. An ultrasound apparatus of a body cavity insertable type comprising:
a handpiece;
a supporting rod that is elongated from the handpiece;
an ultrasound probe that includes a housing that is connected to the supporting rod and an ultrasound transducer that is fixed to the housing; and
an ultrasound transmission medium circulation mechanism that is configured to discharge an ultrasound transmission medium filled in an ultrasound transmission space that is formed at a front of the ultrasound transducer and to inflow the discharged ultrasound transmission medium to the ultrasound transmission space again,
wherein the housing and the ultrasound transducer are configured such that the ultrasound transmission medium filled in the ultrasound transmission space is prevented from inflowing to a rear space of the ultrasound transducer.

2. The ultrasound apparatus of a body cavity insertable type of claim 1, wherein the housing is configured to support entirely an edge of the ultrasound transducer so as to fluidically separate a front space and a rear space of the ultrasound transducer.

3. The ultrasound apparatus of a body cavity insertable type of claim 1, wherein the housing is configured to support entirely lateral edges of the ultrasound transducer and longitudinal edge of the ultrasound transducer are sealed by sealing material, in order to fluidically separate a front surface and a rear surface of the ultrasound transducer.

4. The ultrasound apparatus of a body cavity insertable type of claim 2 or claim 3, wherein lateral edges of the ultrasound transducer are formed to have a concave curved surface shape, and wherein the housing has a supporting portion having a shape corresponding to the concave curved surface of the lateral edge so as to support the lateral edge.

5. The ultrasound apparatus of a body cavity insertable type of claim 1, wherein the ultrasound transmission medium circulation mechanism comprises:
a reservoir storing the ultrasound transmission medium;
a pump that is configured to move the ultrasound transmission medium from the reservoir to the ultrasound transmission space;
an inlet passage forming a passage through which the ultrasound transmission medium discharged from the reservoir inflows into the ultrasound transmission space; and
an outlet passage forming a passage through which the ultrasound transmission medium discharged from the ultrasound transmission space inflows into the reservoir.

6. The ultrasound apparatus of a body cavity insertable type of claim 5, further comprising a sealing member that is disposed between the ultrasound probe and the supporting rod, wherein the inlet passage and the outlet passage pass through the sealing member.

7. The ultrasound apparatus of a body cavity insertable type of claim 6, wherein the inlet passage comprises an inlet conduit passing through the supporting rod and the outlet passage comprises an outlet conduit passing through the supporting rod, and wherein the inlet conduit and the outlet conduit pass through the sealing member.

8. The ultrasound apparatus of a body cavity insertable type of claim 5, wherein the inlet passage comprises an inlet conduit passing through the supporting rod and the outlet passage comprises an outlet conduit passing through the supporting rod, and
wherein the housing comprises:
an inlet connection passage that is connected to the inlet conduit;
an inlet hole that connects the inlet connection passage and the ultrasound transmission space;
an outlet connection passage that is connected to the outlet conduit; and
an outlet hole that connects the outlet connection passage and the ultrasound transmission space.

9. The ultrasound apparatus of a body cavity insertable type of claim 8, wherein the inlet connection passage and the inlet hole are disposed at one side with respect to a longitudinal direction of the hosing, and wherein the outlet connection passage and the outlet hole are disposed at the other side with respect to the longitudinal direction to face respectively the inlet connection hole and the inlet hole.

10. The ultrasound apparatus of a body cavity insertable type of claim 5, wherein the reservoir has a window for checking an amount of the ultrasound transmission medium filled therein.

11. The ultrasound apparatus of a body cavity insertable type of claim 5, wherein the reservoir has an ultrasound transmission medium injection port for replenishing the ultrasound transmission medium and an automatic closing valve that is installed in the ultrasound transmission medium injection port.

12. The ultrasound apparatus of a body cavity insertable type of claim 5, wherein the reservoir is fixedly provided outside the handpiece, wherein the reservoir comprises an inlet through which the ultrasound transmission medium discharged from the ultrasound probe inflows therein and an outlet through which the ultrasound transmission medium to be supplied to the ultrasound probe is discharged, and wherein the outlet is disposed lower than the outlet.

13. The ultrasound apparatus of a body cavity insertable type of claim 5, further comprising a gyro sensor that detects a posture of the handpiece and a controller that controls an operation of the pump based on a signal of the gyro sensor, wherein the reservoir is installed within the handpiece, and wherein the controller controls such that an alarm signal is output or controls to operate the pump so that the ultrasound transmission space is filled with the ultrasound transmission medium.

14. The ultrasound apparatus of a body cavity insertable type of claim 5, further comprising a gyro sensor that detects a posture of the handpiece and a controller that controls an operation of the pump based on a signal of the gyro sensor,
wherein the reservoir is disposed within the handpiece,
wherein the housing comprises an inlet hole through which the ultrasound transmission medium inflows into the ultrasound transmission space and an outlet hole through which the ultrasound transmission medium is discharged from the ultrasound transmission space, and
wherein in case that based on a signal of the gyro sensor, the handpiece is in a position in that the ultrasound transmission medium is full to an outlet of the reservoir and the outlet hole faces upward, the controller controls such that an alarm signal is output or controls to operate the pump so that the ultrasound transmission space is filled with the ultrasound transmission medium to discharge air bubbles remaining in the ultrasound transmission space.

15. The ultrasound apparatus of a body cavity insertable type of claim 5, further comprising a gyro sensor that detects a posture of the handpiece and a controller that controls an operation of the pump based on a signal of the gyro sensor,
wherein the housing comprises an inlet hole through which the ultrasound transmission medium inflows into the ultrasound transmission space and an outlet hole through which the ultrasound transmission medium is discharged from the ultrasound transmission space, and
wherein in case that based on a signal of the gyro sensor, the handpiece is in a position in that the outlet hole faces upward, the controller controls such that an alarm signal is output or controls to operate the pump so that the ultrasound transmission space is filled with the ultrasound transmission medium to discharge air bubbles remaining in the ultrasound transmission space.

16. The ultrasound apparatus of a body cavity insertable type of claim 1, wherein the housing is provided with an ultrasound passing hole through which a generated ultrasound passes, and further comprising a sealing cover that seals the ultrasound passing hole.

17. The ultrasound apparatus of a body cavity insertable type of claim 16, wherein the sealing cover comprises:
a cover member that is configured to be put on the housing and has an ultrasound passing window that is formed at a position corresponding to the ultrasound passing hole; and
an ultrasound penetration film that is attached to the cover member to seal the ultrasound passing hole.
